# EUROPEAN PATENT APPLICATION

(11) **EP 4 039 262 A1**
(43) Date of publication of application: **10.08.2022**
(21) Application number: 20871159.8
(22) Date of filing: 30.09.2020
(51) Int. Cl.: A61K 36/48, A23L 33/105, A61P 1/14, A61K 129/00

(54) **INTESTINAL ENVIRONMENT-IMPROVING AGENT**

(30) Priority: 04.10.2019 JP 2019183809
(71) Applicant: Acacia-No-Ki Co., Ltd., Hatsukaichi-shi, Hiroshima 738-0034 (JP); Hoshi University, Tokyo 142-8501 (JP)
(72) Inventor: KATAOKA, Takeshi, Hatsukaichi-shi, Hiroshima 738-0034 (JP); KAWAJI, Yasuomi, Hatsukaichi-shi, Hiroshima 738-0034 (JP); OGAWA, Sosuke, Hatsukaichi-shi, Hiroshima 738-0034 (JP); KOBAYASHI, Tomoki, Hatsukaichi-shi, Hiroshima 738-0034 (JP); SUGIYAMA, Kiyoshi, Tokyo 142-8501 (JP); IKARASHI, Nobutomo, Tokyo 142-8501 (JP)
(74) Representative: Griffin, Philippa Jane
(86) International application number: PCT/JP2020/037091
(87) International publication number: WO 2021/065987

(57) **Abstract**

An object of the present invention is to provide an intestinal-environment-improving agent. The object can be achieved by an intestinal-environment-improving agent including an acacia bark extract.

## Description

### Technical Field

The present invention relates to an intestinal-environment-improving agent.

### Background Art

It is considered that about 30,000 species of intestinal bacteria numbering around 100 trillion are living in the human intestine, and intestinal bacteria are roughly classified into three groups (beneficial bacteria, harmful bacteria, and opportunistic bacteria). These bacteria interact with each other, which is intricately balanced. The most abundant intestinal bacteria are opportunistic bacteria, the second abundant intestinal bacteria are beneficial bacteria, and the least bacteria are harmful bacteria. Generally, the ideal ratio of these bacteria is maintained to 7 : 2 : 1 (opportunistic bacteria : beneficial bacteria : harmful bacteria). The increasing a proportion of beneficial bacteria in the intestine is important for health. On the other hand, when the number of harmful bacteria increases, the amount of harmful substances produced by the harmful bacteria increases, and various adverse effects appear in the body. Therefore, recently, "probiotics" which are beneficial bacteria that have beneficial effects on health, "prebiotics" which are a source of good for increasing the number of beneficial bacteria in the intestine, and the like have been focused on, and various health benefits are becoming apparent. In addition, sales of health foods and articles of taste focusing on these are increasing.

**As** beneficial bacteria, lactic acid bacteria, *Bifidobacteria*, and the like are known. As harmful bacteria, *Clostridium perfringens*, *Staphylococci*, *Escherichia coli* (toxic strain) and the like are known. As opportunistic bacteria, *Bacteroides*, *E. coli* (non-toxic strain) and the like are known.

### Summary

### Technical Problem

An object of the present invention is to provide an intestinal-environment-improving agent.

### Solution to Problem

**The** inventors conducted extensive studies and as a result, found that an acacia bark extract improves the intestinal environment.

### Advantageous Effects of Invention

According to the present invention, it is possible to provide an intestinal-environment-improving agent.

### Brief Description of Drawings

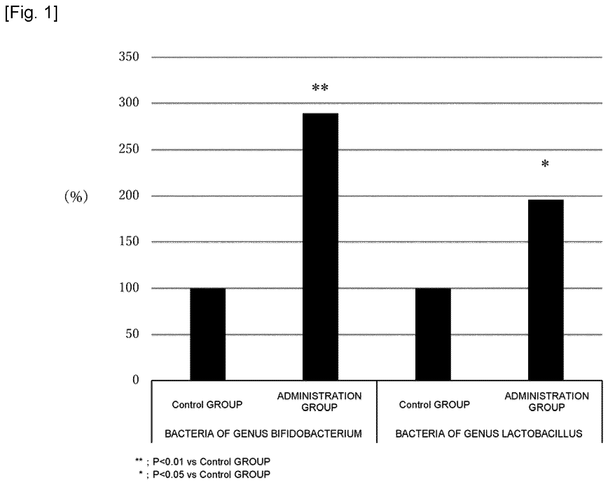

Fig. 1 shows a relationship between administration of an acacia bark extract and the amount of beneficial bacteria in the intestine.

### Description of Embodiments

One embodiment of the present invention relates to an intestinal-environment-improving agent including an acacia bark extract (hereinafter referred to as an "acacia bark extract") as an effective component. In addition, other embodiments of the present invention include, for example, a method of improving the intestinal environment, comprising administering an effective amount of an acacia bark extract to a subject in need thereof, a use of an acacia bark extract for improving the intestinal environment, and a use of an acacia bark extract in production of an agent for improving the intestinal environment.

In this specification, "acacia" means a tree belonging to the genus Acacia. Examples of acacias include Acacia mearnsii De Wild., Acacia mangium Willd., Acacia dealbata Link, Acacia decurrens Willd., and Acacia pycnantha Benth. Although not particularly limited, the acacia is preferably Acacia mearnsii De Wild. or Acacia mangium Willd., and more preferably Acacia mearnsii De Wild.

The methods of preparing and extraction from acacia bark are not particularly restricted, and known methods can be used. For example, methods described in Patent Publication JP-A-2011-51992, Patent Publication JP-A-2010-105923, and Patent Publication JP-A-2009-203209 can be used.

The extraction solvent is not particularly restricted, and examples thereof include water and an organic solvent. The water is preferably hot water. The organic solvent is preferably an alcohol, more preferably an alcohol having 1 to 4 carbon atoms, and particularly preferably ethanol. The extraction solvents may be used alone or two or more thereof may be used in combination.

Although not particularly restricted, it is preferable to perform extraction from the acacia bark with hot water, and additionally perform extraction from the obtained extract with an organic solvent.

The acacia bark extract may be one obtained by purifying the extract extracted by a predetermined method.

The acacia bark extract may contain a plurality of components or may contain only a single component. Examples of components of acacia bark extracts include profisetinidin, prorobinetinidine, procyanidin, prodelphinidin, robinetinidol, fisetinidol, syringic acid, taxifolin, butyne, sucrose, and pinitol.

The intestinal-environment-improving agent can be used as, for example, a food, a drug, a quasi-drug, or an animal feed. Examples of food include food in general and foods with health claims (for example, foods for specified health uses, foods with function claims, and foods with nutrient function claims).

Specific uses of intestinal-environment-improving agents include, for example, increasing the number of beneficial bacteria in the intestine. Examples of beneficial bacteria include lactic acid bacteria and *Bifidobacteria*, and specific examples thereof include bacteria of the genus *Lactobacillus* and bacteria of the genus *Bifidobacterium.*

### Examples

The present invention will be described below in detail with reference to examples, but the technical scope of the present invention is not restricted thereto.

### <Preparation of extract>

The bark of acacia (Acacia mearnsii De Wild.) was crushed and extracted with hot water at 100°C. The obtained solution was spray-dried with a spray dryer to obtain a hot water extract.

### <Intestinal environment improvement test>

### (Experimental animals)

Female BALB/c mice were divided into a control group (n=8) and an administration group (n=8). For 22 days, a normal diet was orally administered to the control group, and a diet containing 3% of an acacia bark extract was orally administered to the administration group. On the 22^{nd} day, feces of the mice from each group were collected.

Here, the mice were bred in a facility at a temperature of 24±1°C and a humidity of 55±5%, and light and dark conditions were set such that light was turned on at 8:00 and turned off at 20:00.

### (Intestinal bacteria analysis)

### 1. DNA extraction and adjustment method

Bacterial DNA was extracted from feces using a QIAamp DNA stool mini kit (Qiagen, Inc). The extraction method was performed according to the protocol included in the QIAamp DNA stool mini kit. The obtained solution was measured at an absorbance of 260 nm and 280 nm with an ultra-micro spectrophotometer, the purity was checked, and the DNA concentration (ng/µL) was calculated.

### 2. Bacteria quantification method

Various primers were prepared, and bacteria of the genus *Bifidobacterium* and bacteria of the genus *Lactobacillus* were determined by real time PCR. 5 µL of SsoAdvanced Universal SYBR Green Supermix, 0.6 µL of a forward primer (5 pmol/µL), 0.6 µL of a reverse primer (5 pmol/µL), 2 µL of a DNA solution, and 2.8 µL of RNase free water were added to each well of a PCR plate. The PCR conditions were set to a denaturation temperature of 95°C for 30 sec, an annealing temperature of 58°C for 30 sec, and an elongation temperature of 72°C for 60 sec. The fluorescence intensity in the amplification procedure was monitored by a CFX Connect Real-Time System (Bio-Rad Laboratories). The expression level was normalized using 16S rRNA.

### 3. Results

The results are shown in Fig. 1. The amount of bacteria of the genus *Bifidobacterium* in the feces in the administration group increased to about 3 times that of the control group. In addition, the amount of bacteria of the genus *Lactobacillus* in the feces in the administration group increased to about 2 times that of the control group.

## Claims

1. An intestinal-environment-improving agent comprising an acacia bark extract.

2. The intestinal-environment-improving agent according to claim 1, wherein improvement of the intestinal environment is an increase in the number of beneficial bacteria in the intestine.

3. The intestinal-environment-improving agent according to claim 2, wherein the beneficial bacteria are lactic acid bacteria and/or *Bifidobacteria.*

4. The intestinal-environment-improving agent according to claim 2, wherein the beneficial bacteria are bacteria of the genus *Lactobacillus* and/or bacteria of the genus *Bifidobacterium.*

5. The intestinal-environment-improving agent according to any one of claims 1 to 4, wherein the acacia is Acacia mearnsii De Wild.

6. The intestinal-environment-improving agent according to any one of claims 1 to 5, wherein the agent is a food.

7. The intestinal-environment-improving agent according to any one of claims 1 to 5, wherein the agent is a drug or a quasi-drug.
